# EUROPEAN PATENT APPLICATION

(11) **EP 3 467 095 A1**
(43) Date of publication of application: **10.04.2019**
(21) Application number: 17802787.6
(22) Date of filing: 23.05.2017
(51) Int. Cl.: C12M 3/00, C08F 230/02, C12N 5/07

(54) **CELL CULTURE VESSEL**

(30) Priority: 27.05.2016 JP 2016106041
(71) Applicant: Nissan Chemical Corporation, Tokyo 103-6119 (JP)
(72) Inventor: HIROI Yoshiomi, Funabashi-shi Chiba 274-0052 (JP); ABE Natsuki, Shiraoka-shi Saitama 349-0294 (JP); NISHINO Taito, Shiraoka-shi Saitama 349-0294 (JP)
(74) Representative: dompatent von Kreisler Selting Werner - Partnerschaft von Patent- und Rechtsanwälten mbB
(86) International application number: PCT/JP2017/019147
(87) International publication number: WO 2017/204201

(57) **Abstract**

The present invention provides: a cell culture vessel characterized in that the surface thereof is coated with a copolymer that comprises a repeat unit containing the group represented by formula (a), a repeat unit containing the group represented by formula (b), and a repeat unit containing the group represented by formula (c); a production method therefor; and a cellular aggregate production method using said cell culture vessel (in the formulae, U^{a1}, U^{a2}, U^{b1}, U^{b2} and U^{b3}, and R^{c} and An⁻ are as defined in the description and the claims).

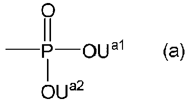

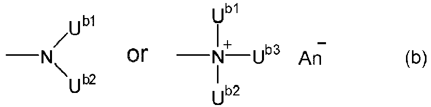

-R^{c} (c)

## Description

### TECHNICAL FIELD

The present invention relates to a cell culture vessel, a method for producing the same, and a method for producing cell aggregates (spheres) using the vessel. In particular, the invention relates to a cell culture vessel characterized in that the surface thereof is coated with a copolymer having a function of inhibiting adhesion of cells, and also relates to a method for producing such vessels.

### BACKGROUND ART

In recent years, there has been a progress in the technique for proliferating or maintaining in-vitro various organs, tissues and cells which play their own functions in animals and plants. The in-vitro proliferation or maintenance of organs and tissues is called organ culture and tissue culture, respectively. The process for the in-vitro proliferation, differentiation or maintenance of cells isolated from organs or tissues is called cell culture. The cell culture is a technique in which isolated cells are proliferated, differentiated or maintained in-vitro in media, and has become indispensable for analyzing in detail the functions and structures of various organs, tissues and cells in the body. The cells and/or tissues cultured by this technique have been used in various fields, including the evaluation of efficacy and toxicity of substances such as chemicals and drugs, the mass production of useful substances such as enzymes, cell growth factors and antibodies, the regenerative medicine for diseased or defective organs, tissues and cells, the breed improvement in plants, and the production of genetically modified crops.

Animal-derived cells are largely divided into two by their properties: suspended cells and adherent cells. The suspended cells are cells that grow or proliferate without scaffolds, while the adherent cells require scaffolds for growth or proliferation. The majority of the cells constituting a living body are the latter adherent cells. Some known methods for culturing adherent cells are monolayer culture, dispersion culture, embedded culture, microcarrier culture, and cell aggregate (sphere) culture.

Particularly in recent years, the development in the regenerative medicine area has drawn attention to the sphere culture in which cells are cultured in an environment more similar to that in-vivo, and a variety of medium compositions and medium additives suited for this culture have been reported (see, for example, Patent Literatures 1 and 2). In the sphere culture, stimuli from culture vessels (substrates) are considered to be an important factor affecting the results of the culture. Thus, cells (in particular, cell aggregates) are to be cultured in a three dimensional environment or in a completely suspended state without stimuli from the culture vessel (see, for example, Patent Literature 3).

The inventors of the present invention carried out studies focusing on phosphate group-containing polymers expected as coating materials having a function of inhibiting adhesion of various biomaterials. As a result, the present inventors have reported that a cell culture vessel which is at least partly coated with a copolymer containing specific anionic group and cationic group inhibits adhesion of cells to the surface (inner surface) of the vessel as well as the coating can be firmly fixed onto the surface of the vessel, so that it is useful as a cell culture vessel improved in elution of the coating into the culture fluid and radiation resistance (see, for example, Patent Literature 4).

### CITATION LIST

### Patent Literature

Patent Literature 1: WO 2014/017513
Patent Literature 2: WO 2013/144372
Patent Literature 3: Japanese Patent Application Kokai Publication No. 2008-61609
Patent Literature 4: WO 2014/0196652

### SUMMARY OF INVENTION

### Technical Problem

An object of the invention is to provide a cell culture vessel, a method for producing the same, and a method for producing cell aggregates using the vessel. In particular, the invention has an object of providing a cell culture vessel characterized in that the surface thereof is coated with a copolymer having a function of inhibiting adhesion of cells, a method for producing such vessels, and a method for producing cell aggregates using the vessel.

In the culturing of cells (in particular, cell aggregates) in a three dimensional environment or in a completely suspended state, the conventional problems are the adhesion of cells to the vessel surface, and the elution of a coating on the cell culture vessel into the culture fluid. Another problem is that proteins produced or secreted by cell culture or proteins contained in media adhere to the vessel surface. While cell culture vessels coated with a hydrophilic compound can improve the adhesion of cells to the vessel surface, problems remain in that the coating on the cell culture vessel is eluted into the culture fluid, or proteins adhere to the vessel surface.

### Solution to Problem

The present inventors further carried out extensive studies focusing on polymers having a phosphate group. As a result, the present inventors have found that a cell culture vessel coated, on at least a portion of the surface thereof, with a copolymer containing specific anionic and cationic groups and also a specific hydrophobic group, inhibits the adhesion of not only cells but also proteins to the vessel surface, and is also useful in that the coating is firmly fixed onto the vessel surface and thus negligibly eluted into a culture fluid. The present invention has been completed based on the finding.

Specifically, aspects of the present invention reside in the following.
1. A cell culture vessel having a surface coated with a copolymer comprising a repeating unit containing a group represented by the following formula (a), a repeating unit containing a group represented by the following formula (b), and a repeating unit containing a group represented by the following formula (c):

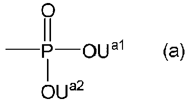

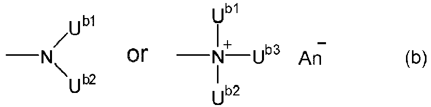

   -R^{c} (c)

   wherein
   U^{a1}, U^{a2}, U^{b1}, U^{b2} and U^{b3} are each independently a hydrogen atom or a C₁₋₅ linear or branched alkyl group;
   R^{c} is a C₁₋₁₈ linear or branched alkyl group, a C₃₋₁₀ alicyclic hydrocarbon group, a C₆₋₁₀ aryl group, a C₇₋₁₅ aralkyl group or a C₇₋₁₅ aryloxyalkyl group (wherein the aryl moiety may be substituted with an optionally halogenated C₁₋₅ linear or branched alkyl group); and
   An⁻ is an anion selected from the group consisting of halide ion, inorganic acid ion, hydroxide ion and isothiocyanate ion.
2. The cell culture vessel described in 1, wherein the repeating units containing groups represented by the formulae (a), (b) and (c) are repeating units derived from monomers represented by the following formulae (A), (B) and (C), respectively: 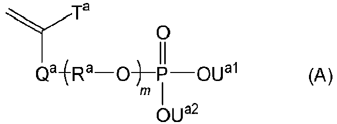 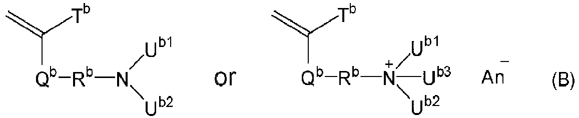 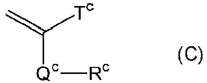 wherein
   T^{a}, T^{b}, T^{c}, U^{a1}, U^{a2}, U^{b1}, U^{b2} and U^{b3} are each independently a hydrogen atom or a C₁₋₅ linear or branched alkyl group;
   Q^{a} and Q^{b} are each independently a single bond, an ester bond or an amide bond, Q^{c} is a single bond, an ether bond or an ester bond;
   R^{a} and R^{b} are each independently an optionally halogenated C₁₋₁₀ linear or branched alkylene group, R^{c} is a C₁₋₁₈ linear or branched alkyl group, a C₃₋₁₀ alicyclic hydrocarbon group, a C₆₋₁₀ aryl group, a C₇₋₁₅ aralkyl group or a C₇₋₁₅ aryloxyalkyl group (wherein the aryl moiety may be substituted with an optionally halogenated C₁₋₅ linear or branched alkyl group);
   An⁻ is an anion selected from the group consisting of halide ion, inorganic acid ion, hydroxide ion and isothiocyanate ion; and
   m is an integer of 0 to 6.
3. The cell culture vessel described in 2, wherein m is 1, and R^{a} and R^{b} are each independently an ethylene group or a propylene group.
4. The cell culture vessel described in any one of 1 to 3, wherein the copolymer further comprises a crosslinked structure derived from a monomer represented by the following formula (D) or (E): 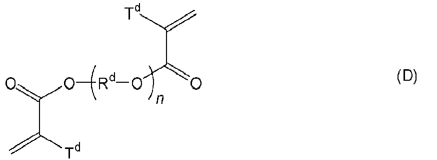 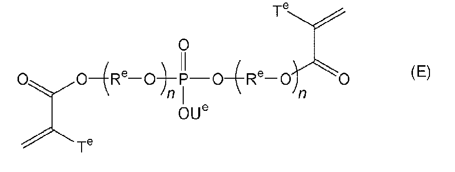 wherein
   T^{d}, T^{e} and U^{e} are each independently a hydrogen atom or a C₁₋₅ linear or branched alkyl group;
   R^{d} and R^{e} are each independently an optionally halogenated C₁₋₁₀ linear or branched alkylene group; and
   n is an integer of 1 to 6.
5. The cell culture vessel described in 4, wherein T^{d} and T^{e} are each independently a hydrogen atom or a methyl group, U^{e} is a hydrogen atom, and R^{d} and R^{e} are each independently an ethylene group or a propylene group.
6. The cell culture vessel described in any one of 1 to 5, which has a function of inhibiting adhesion of proteins.
7. A method for producing a cell culture vessel, comprising a step of coating at least a portion of the surface of a cell culture vessel with a copolymer comprising a repeating unit containing a group represented by the following formula (a), a repeating unit containing a group represented by the following formula (b), and a repeating unit containing a group represented by the following formula (c):

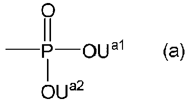

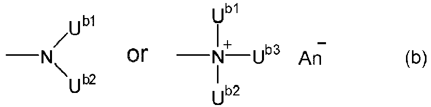

   -R^{c} (c)

   wherein
   U^{a1}, U^{a2}, U^{b1}, U^{b2} and U^{b3} are each independently a hydrogen atom or a C₁₋₅ linear or branched alkyl group;
   R^{c} is a C₁₋₁₈ linear or branched alkyl group, a C₃₋₁₀ alicyclic hydrocarbon group, a C₆₋₁₀ aryl group, a C₇₋₁₅ aralkyl group or a C₇₋₁₅ aryloxyalkyl group (wherein the aryl moiety may be substituted with an optionally halogenated C₁₋₅ linear or branched alkyl group); and
   An⁻ is an anion selected from the group consisting of halide ion, inorganic acid ion, hydroxide ion and isothiocyanate ion.
8. The production method described in 7, wherein the repeating units containing groups represented by the formulae (a), (b) and (c) are repeating units derived from monomers represented by the following formulae (A), (B) and (C), respectively: 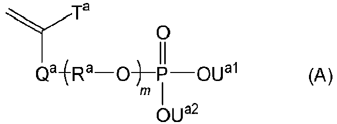 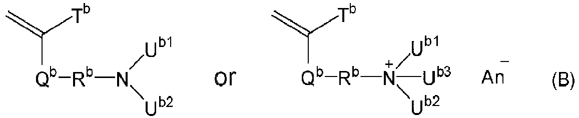 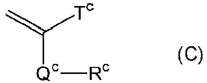 wherein
   T^{a}, T^{b}, T^{c}, U^{a1}, U^{a2}, U^{b1}, U^{b2} and U^{b3} are each independently a hydrogen atom or a C₁₋₅ linear or branched alkyl group;
   Q^{a} and Q^{b} are each independently a single bond, an ester bond or an amide bond, Q^{c} is a single bond, an ether bond or an ester bond;
   R^{a} and R^{b} are each independently an optionally halogenated C₁₋₁₀ linear or branched alkylene group, R^{c} is a C₁₋₁₈ linear or branched alkyl group, a C₃₋₁₀ alicyclic hydrocarbon group, a C₆₋₁₀ aryl group, a C₇₋₁₅ aralkyl group or a C₇₋₁₅ aryloxyalkyl group (wherein the aryl moiety may be substituted with an optionally halogenated C₁₋₅ linear or branched alkyl group);
   An⁻ is an anion selected from the group consisting of halide ion, inorganic acid ion, hydroxide ion and isothiocyanate ion; and
   m is an integer of 0 to 6.
9. The production method described in 7 or 8, wherein the coating step is performed using a varnish including the copolymer.
10. The production method described in 9, wherein the pH of the varnish including the copolymer is adjusted beforehand.
11. The production method described in any one of 7 to 10, further comprising a step of washing the cell culture vessel coated, before and/or after a drying step.
12. The production method described in 11, wherein the washing after the drying step is performed using at least one solvent selected from the group consisting of water and aqueous electrolyte solutions.
13. A method for producing a cell aggregate, characterized by using the cell culture vessel described in any one of 1 to 6, or a cell culture vessel produced by the production method described in any one of 7 to 12.

### Advantageous Effects of Invention

The cell culture vessels of the present invention can inhibit the adhesion of cells and proteins to the vessel surface by virtue of at least a portion of the surface being coated with a copolymer including an anion represented by the formula (a), a cation represented by the formula (b) and a hydrophobic group represented by the formula (c). The adhesion of cells and proteins can be prevented probably because the surface of the vessel is kept electrically neutral due to the electrostatic balance between the cation and the anion. At the same time, the cation and the anion in the coating form an ion bond (an ion complex) so as to establish an adhesion to any types of vessel bases such as glass, fibers, inorganic particles and resins (synthetic resins and natural resins). Further, after being formed, the coating is highly durable in contact with aqueous solvents (such as water, phosphate buffered saline (PBS) and alcohols).

Further, as a result of the introduction of a hydrophobic group represented by the formula (c) into the copolymer, the coating is enhanced in the ability to inhibit adhesion of proteins, and also attains good fixing to resins such as plastics and further, after fixing, it becomes a coating excellent in durability against an aqueous solvent.

That is, the cell culture vessels provided by the present invention can inhibit adhesion of not only cells but also proteins to the vessel surface and have high durability in contact with solvents.

### BRIEF DESCRIPTION OF DRAWING

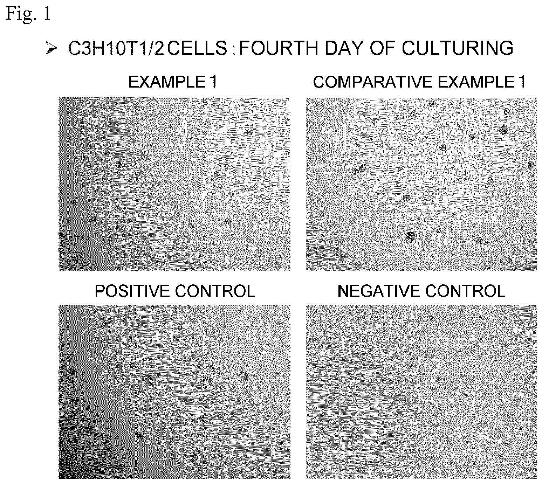

Fig. 1 shows the results of an inverted microscope observation of cell adhesion to plates of Example 1, Comparative Example 1, negative control and positive control, after 4 days of culture.

### Description of Embodiments

### 1. Description of terms

The terms used in the present invention have the following definitions unless otherwise specified.

In the present invention, the "halogen atom" means fluorine atom, chlorine atom, bromine atom or iodine atom.

In the present invention, the "alkyl group" means linear or branched, monovalent saturated aliphatic hydrocarbon group. Examples of the "C₁₋₅ linear or branched alkyl groups" include methyl group, ethyl group, n-propyl group, isopropyl group, n-butyl group, isobutyl group, s-butyl group, t-butyl group, n-pentyl group, 1-methylbutyl group, 2-methylbutyl group, 3-methylbutyl group, 1,1-dimethylpropyl group, 1,2-dimethylpropyl group, 2,2-dimethylpropyl group and 1-ethylpropyl group. Examples of the "C₁₋₁₈ linear or branched alkyl groups" include the C₁₋₅ linear or branched alkyl groups mentioned above, and further include hexyl group, heptyl group, octyl group, nonyl group, decyl group, undecyl group, dodecyl group, tridecyl group, tetradecyl group, pentadecyl group, hexadecyl group, heptadecyl group, octadecyl group, and isomers thereof.

In the present invention, the "optionally halogenated C₁₋₅ linear or branched alkyl group" means any of the above-mentioned C₁₋₅ linear or branched alkyl groups, or any of the above-mentioned C₁₋₅ linear or branched alkyl groups that is substituted with one or more of the above-mentioned halogen atoms. Examples of the "C₁₋₅ linear or branched alkyl groups" are as mentioned above. The "C₁₋₅ linear or branched alkyl group substituted with one or more halogen atoms" means any of the above-mentioned C₁₋₅ linear or branched alkyl groups that is substituted with a halogen atom in place of one or more hydrogen atoms, with examples including fluoromethyl group, difluoromethyl group, trifluoromethyl group, chloromethyl group, dichloromethyl group, trichloromethyl group, bromomethyl group, iodomethyl group, 2,2,2-trifluoroethyl group, 2,2,2-trichloroethyl group, perfluoroethyl group, perfluorobutyl group and perfluoropentyl group.

In the present invention, the "ester bond" means -C(=O)-O- or -O-C(=O)-, the "amide bond" means -NHC(=O)- or -C(=O)NH-, and the ether bond means -O-.

In the present invention, the "optionally halogenated C₁₋₁₀ linear or branched alkylene group" means a C₁₋₁₀ linear or branched alkylene group, or a C₁₋₁₀ linear or branched alkylene group substituted with one or more halogen atoms. Here, the "alkylene group" means a divalent organic group corresponding to the alkyl group described hereinabove. Examples of the "C₁₋₁₀ linear or branched alkylene groups" include methylene group, ethylene group, propylene group, trimethylene group, tetramethylene group, 1-methylpropylene group, 2-methylpropylene group, dimethylethylene group, ethylethylene group, pentamethylene group, 1-methyl-tetramethylene group, 2-methyl-tetramethylene group, 1,1-dimethyl-trimethylene group, 1,2-dimethyl-trimethylene group, 2,2-dimethyl-trimethylene group, 1-ethyl-trimethylene group, hexamethylene group, octamethylene group and decamethylene group. Of these, ethylene group, propylene group, octamethylene group and decamethylene group are preferable. C₁₋₅ linear or branched alkylene groups such as, for example, ethylene group, propylene group, trimethylene group and tetramethylene group are more preferable. Ethylene group and propylene group are particularly preferable. The "C₁₋₁₀ linear or branched alkylene group substituted with one or more halogen atoms" means any of the above-mentioned alkylene groups that is substituted with a halogen atom in place of one or more hydrogen atoms, and, in particular, ethylene or propylene group substituted with a halogen atom in place of part or all of the hydrogen atoms are preferable.

In the present invention, the "C₃₋₁₀ alicyclic hydrocarbon group" means a C₃₋₁₀ monocyclic or polycyclic, saturated or partially unsaturated, monovalent aliphatic hydrocarbon group. Of such groups, C₃₋₁₀ monocyclic or bicyclic, saturated monovalent aliphatic hydrocarbon groups are preferable, with examples including C₃₋₁₀ cycloalkyl groups such as cyclopropyl group, cyclobutyl group and cyclohexyl group, and C₄₋₁₀ bicycloalkyl groups such as bicyclo[3.2.1]octyl group, bornyl group and isobornyl group.

In the present invention, the "C₆₋₁₀ aryl group" means a C₆₋₁₀ monocyclic or polycyclic, monovalent aromatic hydrocarbon group, with examples including phenyl group, naphthyl group and anthryl group. The "C₆₋₁₀ aryl group" may be substituted with one or more of the above-mentioned "optionally halogenated C₁₋₅ linear or branched alkyl groups".

In the present invention, the "C₇₋₁₅ aralkyl group" means group -R-R' wherein R represents the "C₁₋₅ alkylene group" described hereinabove, and R' represents the "C₆₋₁₀ aryl group" described hereinabove, with examples including benzyl group, phenethyl group and α-methylbenzyl group. The aryl moiety of the "C₇₋₁₅ aralkyl group" may be substituted with one or more of the "optionally halogenated C₁₋₅ linear or branched alkyl groups" described hereinabove.

In the present invention, the "C₇₋₁₅ aryloxyalkyl group" means group -R-O-R' wherein R represents the "C₁₋₅ alkylene group" described hereinabove, and R' represents the "C₆₋₁₀ aryl group" described hereinabove, with examples including phenoxymethyl group, phenoxyethyl group and phenoxypropyl group. The aryl moiety of the "C₇₋₁₅ aryloxyalkyl group" may be substituted with one or more of the "optionally halogenated C₁₋₅ linear or branched alkyl groups" described hereinabove.

In the present invention, the "halide ion" means fluoride ion, chloride ion, bromide ion or iodide ion.

In the present invention, the "inorganic acid ion" means carbonate ion, sulfate ion, phosphate ion, hydrogenphosphate ion, dihydrogenphosphate ion, nitrate ion, perchlorate ion or borate ion.

The anion An⁻ is preferably a halide ion, a sulfate ion, a phosphate ion, a hydroxide ion or an isothiocyanate ion, and particularly preferably a halide ion.

In the present invention, the (meth)acrylate compounds mean both acrylate compounds and methacrylate compounds. For example, (meth)acrylic acid means acrylic acid and methacrylic acid.

For example, the phrase "having a function of inhibiting adhesion of cells" means that the relative absorbance (WST O. D. 450 nm) (%) is not more than 50%, preferably not more than 30%, and more preferably not more than 20% in comparison with the absorbance in the absence of a coating film as measured by the method described in Examples with a cell counting reagent, (Absorbance (WST O. D. 450 nm) in Example/Absorbance (WST O. D. 450 nm) in Comparative Example x 100).

For example, the phrase "having a function of inhibiting adhesion of proteins" means that the relative mass (%) per unit area is not more than 50%, preferably not more than 30%, and more preferably not more than 20% in comparison with the mass in the absence of a coating film as determined by QCM-D measurement by the method described in Examples, (Mass per unit area (ng/cm²) in Example/Mass per unit area (ng/cm²) in Comparative Example x 100).

Examples of the proteins include fibrinogen, bovine serum albumin (BSA), human albumin, various globulins, β-lipoprotein, various antibodies (IgG, IgA, IgM), peroxidase, various complements, various lectins, fibronectin, lysozyme, von Willebrand factor (vWF), serum γ-globulin, pepsin, ovalbumin, insulin, histone, ribonuclease, collagen and cytochrome c. The cell culture vessel of present invention has a function of inhibiting adhesion of proteins contained in serum (albumin and globulin).

### 2. Description of Invention

### 〈〈Cell culture vessels〉〉

The first aspect of the present invention resides in a cell culture vessel characterized in that at least a portion of the surface is coated with a copolymer including a repeating unit containing a group represented by the following formula (a), a repeating unit containing a group represented by the following formula (b), and a repeating unit containing a group represented by the following formula (c):

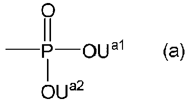

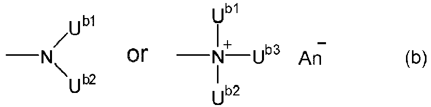

-R^{c} (c)

In the formulae,
U^{a1}, U^{a2}, U^{b1}, U^{b2} and U^{b3} are each independently a hydrogen atom or a C₁₋₅ linear or branched alkyl group;
R^{c} is a C₁₋₁₈ linear or branched alkyl group, a C₃₋₁₀ alicyclic hydrocarbon group, a C₆₋₁₀ aryl group, a C₇₋₁₅ aralkyl group or a C₇₋₁₅ aryloxyalkyl group (wherein the aryl moiety may be substituted with an optionally halogenated C₁₋₅ linear or branched alkyl group); and
An⁻ is an anion selected from the group consisting of halide ion, inorganic acid ion, hydroxide ion and isothiocyanate ion.

### 〈Cells〉

The cells in the invention are the most basic units that constitute an animal or a plant. The elements of a cell are cytoplasm and various organelles enclosed within a membrane. The cells may or may not contain a nucleus containing DNA. Examples of the animal cells in the invention include germ cells such as sperms and eggs, somatic cells constituting a living body, stem cells (such as pluripotent stem cells), progenitor cells, cancer cells isolated from a living body, cells (cell lines) isolated from a living body and immortalized to exist stably in-vitro, cells isolated from a living body and artificially genetically modified, and cells isolated from a living body and artificially nuclear exchanged. Examples of the somatic cells constituting a living body include, but are not limited to, fibroblasts, bone marrow cells, B lymphocytes, T lymphocytes, neutrophils, red blood cells, platelets, macrophages, monocytes, bone cells, bone marrow cells, pericytes, dendritic cells, keratinocytes, fat cells, mesenchymal cells, epithelial cells, epidermal cells, endothelial cells, vascular endothelial cells, hepatic parenchymal cells, cartilage cells, cumulus cells, neural cells, glial cells, neurons, oligodendrocyte, microglia, astroglial cells, heart cells, esophagus cells, muscle cells (for example, smooth muscle cells and skeletal muscle cells), pancreatic beta cells, melanocytes, hematopoietic precursor cells (for example, CD34 positive cord blood-derived cells), and mononuclear cells. For example, the somatic cells include cells collected from any tissues such as skin, kidney, spleen, adrenal gland, liver, lung, ovary, pancreas, uterus, stomach, colon, small intestine, large intestine, bladder, prostate, testis, thymus, muscle, connective tissues, bone, cartilage, blood vessel tissues, blood (including umbilical cord blood), bone marrow, heart, eye, brain and neural tissues. The stem cells are cells that can divide to replicate themselves, and can differentiate into other types of cells, with examples including, but being not limited to, embryonic stem cells (ES cells), embryonic tumor cells, embryonic germ stem cells, induced pluripotent stem cells (iPS cells), neural stem cells, hematopoietic stem cells, mesenchymal stem cells, liver stem cells, pancreatic stem cells, muscle stem cells, germ stem cells, intestinal stem cells, cancer stem cells and hair follicle stem cells. Of the stem cells described above, ES cells, embryonic germ stem cells and iPS cells are example pluripotent stem cells. The progenitor cells are the stem cells in the course of the process of differentiation into specific somatic cells or germ cells. The cancer cells are cells that are derived from somatic cells and possess unlimited proliferative potential. The cell lines are cells that have acquired unlimited proliferation ability by artificial in vitro manipulation.

Examples of the cancer cell lines include, but are not limited to, human breast cancer cell lines such as HBC-4, BSY-1, BSY-2, MCF-7, MCF-7/ADR RES, HS578T, MDA-MB-231, MDA-MB-435, MDA-N, BT-549 and T47D, human cervical cancer cell lines such as HeLa, human lung cancer cell lines such as A549, EKVX, HOP-62, HOP-92, NCI-H23, NCI-H226, NCI-H322M, NCI-H460, NCI-H522, DMS273 and DMS114, human colorectal cancer cell lines such as Caco-2, COLO-205, HCC-2998, HCT-15, HCT-116, HT-29, KM-12, SW-620 and WiDr, human prostate cancer cell lines such as DU-145, PC-3 and LNCaP, human central nervous system cancer cell lines such as U251, SF-295, SF-539, SF-268, SNB-75, SNB-78 and SNB-19, human ovarian cancer cell lines such as OVCAR-3, OVCAR-4, OVCAR-5, OVCAR-8, SK-OV-3 and IGROV-1, human kidney cancer cell lines such as RXF-631L, ACHN, UO-31, SN-12C, A498, CAKI-1, RXF-393L, 786-0 and TK-10, human stomach cancer cell lines such as MKN45, MKN28, St-4, MKN-1, MKN-7 and MKN-74, skin cancer cell lines such as LOX-IMVI, LOX, MALME-3M, SK-MEL-2, SK-MEL-5, SK-MEL-28, UACC-62, UACC-257 and M14, and leukemia cell lines such as CCRF-CRM, K562, MOLT-4, HL-60TB, RPMI8226, SR, UT7/TPO and Jurkat. Examples of the cell lines include, but are not limited to, HEK293 (human embryonic kidney cells), MDCK, MDBK, BHK, C-33A, AE-1, 3D9, Ns0/1, NIH3T3, PC12, S2, Sf9, Sf21, High Five (registered trademark) and Vero. Examples of the hepatic cell lines include, but are not limited to, HepG2, Hep3B, HepaRG (registered trademark), JHH7, HLF, HLE, PLC/PRF/5, WRL68, HB611, SK-HEP-1, HuH-4 and HuH-7.

The plant-derived cells in the present invention include cells isolated from tissues of plants, and also include protoplasts which are the cells having the cell walls artificially removed.

### 〈Vessels〉

The vessel which constitutes the cell culture vessel of the invention by being coated with the copolymer may be any type of a vessel which can be used in the technical field of interest. Examples thereof include vessels generally used for the culturing of cells, including cylindrical dishes, flasks, plastic bags, Teflon (registered trademark) bags, dishes, petri dishes, tissue culture dishes, multidishes, microplates, microwell plates, multiplates, multiwell plates, chamber slides, cell culture flasks, spinner flasks, tubes, trays, culture bags and roller bottles. Some preferred vessels are 6-1536 well multiwell plates and petri dishes.

Typical materials of the vessels are glass and resins. From points of view such as processability and economic efficiency, resins are preferably used. The resins may be natural resins or synthetic resins. Examples of the natural resins include cellulose, cellulose triacetate (CTA) and dextran sulfate immobilized cellulose. Examples of the synthetic resins include polyacrylonitrile (PAN), polyester-based polymer alloy (PEPA), polystyrene (PS), polysulfone (PSF), polyethylene terephthalate (PET), polymethyl methacrylate (PMMA), polyvinyl alcohol (PVA), polyurethane (PU), ethylene vinyl alcohol (EVAL), polyethylene (PE), polyester, polypropylene (PP), polyvinylidene fluoride (PVDF), polyethersulfone (PES), polycarbonate (PC), polyvinyl chloride (PVC), polytetrafluoroethylene (PTFE), ultrahigh molecular weight polyethylene (UHPE), polydimethylsiloxane (PDMS), acrylonitrile-butadiene-styrene resin (ABS), Teflon (registered trademark), cycloolefin polymers (COP) (for example, ZEONOR (registered trademark) and ZEONEX (registered trademark) (manufactured by ZEON CORPORATION)), and various ion exchange resins. In the production of the cell culture vessels of the invention, the copolymer can be applied to coat at least a portion of the surface of the vessel without the need of high-temperature treatment. Thus, application is possible to vessels having low heat resistance such as resins.

### 〈Copolymers〉

The cell culture vessel of the present invention is characterized in that at least a portion of the surface of the vessel is coated with the specific copolymer. The copolymer of the present invention includes a repeating unit containing a group represented by the following formula (a), a repeating unit containing a group represented by the following formula (b), and a repeating unit containing a group represented by the following formula (c):

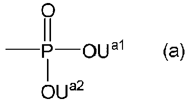

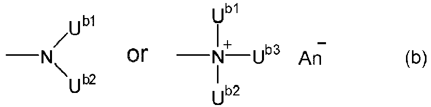

-R^{c} (c)

In the formulae,
U^{a1}, U^{a2}, U^{b1}, U^{b2} and U^{b3} are each independently a hydrogen atom or a C₁₋₅ linear or branched alkyl group;
R^{c} is a C₁₋₁₈ linear or branched alkyl group, a C₃₋₁₀ alicyclic hydrocarbon group, a C₆₋₁₀ aryl group, a C₇₋₁₅ aralkyl group or a C₇₋₁₅ aryloxyalkyl group (wherein the aryl moiety may be substituted with an optionally halogenated C₁₋₅ linear or branched alkyl group); and
An⁻ is an anion selected from the group consisting of halide ion, inorganic acid ion, hydroxide ion and isothiocyanate ion.

The copolymer of the invention is not particularly limited as long as the copolymer includes a repeating unit containing a group represented by the above formula (a), a repeating unit containing a group represented by the above formula (b), and a repeating unit containing a group represented by the above formula (c). In the present invention, the repeating unit containing a group represented by the above formula (c) is different from the repeating unit containing a group represented by the above formula (a), and from the repeating unit containing a group represented by the above formula (b). The copolymer is preferably one obtained by the radical polymerization of a monomer containing a group represented by the above formula (a), a monomer containing a group represented by the above formula (b), and a monomer containing a group represented by the above formula (c). Polymers obtained by polycondensation or polyaddition reaction may be also used. Examples of the copolymers include vinyl polymerized polymers obtained by the reaction of olefins, and also include polyamides, polyesters, polycarbonates and polyurethanes. Of these, vinyl polymerized polymers obtained by the reaction of olefins, or (meth)acrylate polymers obtained by the polymerization of (meth)acrylate compounds are particularly preferable.

Preferably, the monomers containing groups represented by the formulae (a), (b) and (c) are monomers represented by the following formulae (A), (B) and (C), respectively:

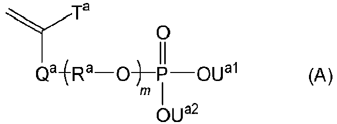

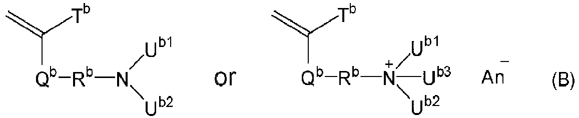

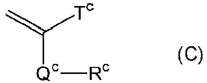

In the formulae,
T^{a}, T^{b}, T^{c}, U^{a1}, U^{a2}, U^{b1}, U^{b2} and U^{b3} are each independently a hydrogen atom or a C₁₋₅ linear or branched alkyl group;
Q^{a} and Q^{b} are each independently a single bond, an ester bond or an amide bond, Q^{c} is a single bond, an ether bond or an ester bond;
R^{a} and R^{b} are each independently an optionally halogenated C₁₋₁₀ linear or branched alkylene group, R^{c} is a C₁₋₁₈ linear or branched alkyl group, a C₃₋₁₀ alicyclic hydrocarbon group, a C₆₋₁₀ aryl group, a C₇₋₁₅ aralkyl group or a C₇₋₁₅ aryloxyalkyl group (wherein the aryl moiety may be substituted with an optionally halogenated C₁₋₅ linear or branched alkyl group);
An⁻ is an anion selected from the group consisting of halide ion, inorganic acid ion, hydroxide ion and isothiocyanate ion; and
m is an integer of 0 to 6. Therefore, the repeating units derived from the monomers of the formulae (A), (B) and (C) are represented by the following formulae (a1), (b1) and (c1), respectively:

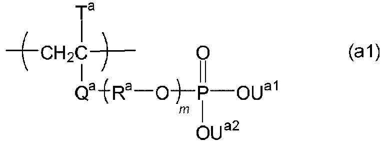

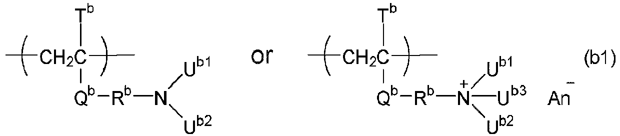

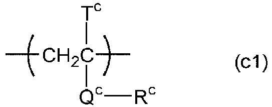

In the formulae, T^{a}, T^{b}, T^{c}, U^{a1}, U^{a2}, U^{b1}, U^{b2}, U^{b3}, Q^{a}, Q^{b}, Q^{c}, R^{a}, R^{b}, R^{c}, An⁻ and m are the same as defined hereinabove.

T^{a}, T^{b} and T^{c} are preferably each independently a hydrogen atom, a methyl group or an ethyl group, and more preferably each independently a hydrogen atom or a methyl group.

U^{a1}, U^{a2}, U^{b1}, U^{b2} and U^{b3} are preferably each independently a hydrogen atom, a methyl group, an ethyl group or a t-butyl group. U^{a1} and U^{a2} are more preferably each a hydrogen atom. U^{b1} and U^{b2} (and U^{b3}) are more preferably each a methyl group or an ethyl group, and particularly preferably each a methyl group.

Q^{a} and Q^{b} are preferably each independently an ester bond (-C(=O)-O- or -O-C(=O)-) or an amide bond (-NHC(=O)- or -C(=O)NH-), more preferably each independently -C(=O)-O- or -C(=O)NH-, and particularly preferably -C(=O)-O-. Q^{C} is preferably an ether bond or an ester bond (-C(=O)-O- or -O-C(=O)-), more preferably -O- or -C(=O)-O-, and particularly preferably -C(=O)-O-.

R^{a} and R^{b} are preferably each independently an optionally halogenated C₁₋₃ linear or branched alkylene group, and are more preferably each independently an ethylene or propylene group or an ethylene or propylene group substituted with one chlorine atom, and are particularly preferably each an ethylene group or a propylene group. R^{c} is preferably a C₄₋₈ linear or branched alkyl group or a C₃₋₈ alicyclic hydrocarbon group, more preferably a C₄₋₆ linear or branched alkyl group or a C₃₋₆ alicyclic hydrocarbon group, and particularly preferably a butyl group or a cyclohexyl group.

The anion An⁻ is preferably a halide ion, a sulfate ion, a phosphate ion, a hydroxide ion or an isothiocyanate ion, and particularly preferably a halide ion.

In the formulae (A) and (B), m is preferably an integer of 0 to 3, more preferably an integer of 1 or 2, and particularly preferably 1.

Specific examples of the formula (A) described above include vinylphosphonic acid, acid phosphoxyethyl (meth)acrylate, 3-chloro-2-acid phosphoxypropyl (meth)acrylate, acid phosphoxypropyl (meth)acrylate, acid phosphoxymethyl (meth)acrylate, acid phosphoxypolyoxyethylene glycol mono(meth)acrylate and acid phosphoxypolyoxypropylene glycol mono(meth)acrylate. Of these, vinylphosphonic acid and acid phosphoxyethyl methacrylate (= 2-(methacryloyloxy)ethyl phosphate) are preferably used.

The structures of vinylphosphonic acid, acid phosphoxyethyl methacrylate (= 2-(methacryloyloxy)ethyl phosphate), acid phosphoxypolyoxyethylene glycol monomethacrylate and acid phosphoxypolyoxypropylene glycol monomethacrylate are represented by the following formulae (A-1) to (A-4), respectively.

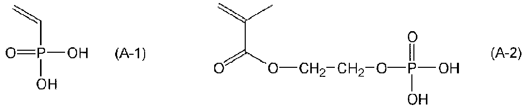

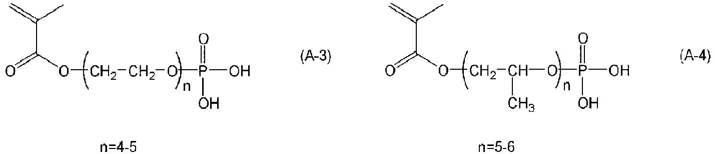

These compounds sometimes contain (meth)acrylate compounds with two functional groups of the general formula (D) or (E) described later which are formed during the synthesis.

Specific examples of the formula (B) described above include dimethylaminoethyl (meth)acrylate, diethylaminoethyl (meth)acrylate, dimethylaminopropyl (meth)acrylate, 2-(t-butylamino)ethyl (meth)acrylate and methacroylcholine chloride. Of these, dimethylaminoethyl (meth)acrylate, methacroylcholine chloride and 2-(t-butylamino)ethyl (meth)acrylate are preferably used.

The structures of dimethylaminoethyl acrylate (= acrylic acid 2-(dimethylamino)ethyl), diethylaminoethyl methacrylate (= methacrylic acid 2-(diethylamino)ethyl), dimethylaminoethyl methacrylate (= methacrylic acid 2-(dimethylamino)ethyl), methacroylcholine chloride and 2-(t-butylamino)ethyl methacrylate (= methacrylic acid 2-(t-butylamino)ethyl) are represented by the following formulae (B-1) to (B-5), respectively.

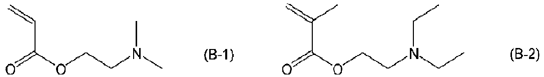

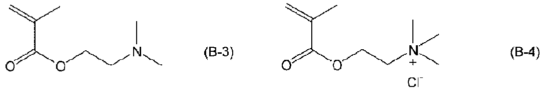

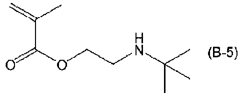

Specific examples of the formula (C) described above include linear or branched alkyl esters of (meth)acrylic acid such as butyl (meth)acrylate, 2-ethylhexyl (meth)acrylate, lauryl (meth)acrylate and stearyl (meth)acrylate; cyclic alkyl esters of (meth)acrylic acid such as cyclohexyl (meth)acrylate and isobornyl (meth)acrylate; aralkyl esters of (meth)acrylic acid such as benzyl (meth)acrylate and phenethyl (meth)acrylate; styrene monomers such as styrene, methylstyrene and chloromethylstyrene; vinyl ether monomers such as methyl vinyl ether and butyl vinyl ether; and vinyl ester monomers such as vinyl acetate and vinyl propionate. Of these, butyl (meth)acrylate and cyclohexyl (meth)acrylate are preferably used.

The structures of butyl methacrylate (= methacrylic acid butyl) and cyclohexyl methacrylate (= methacrylic acid cyclohexyl) are represented by the following formulae (C-1) and (C-2), respectively.

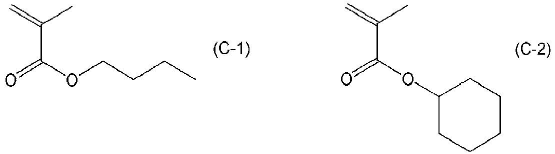

In the copolymer of the invention, the proportion of the repeating units containing a group represented by the formula (a) (or the repeating units represented by the formula (a1)) is 3 mol% to 80 mol%, preferably 3.5 mol% to 50 mol%, and more preferably 4 mol% to 40 mol%. The copolymer of the invention may include two or more kinds of repeating units containing a group represented by the formula (a) (or repeating units represented by the formula (a1)).

In the copolymer of the invention, the proportion of the repeating units containing a group represented by the formula (b) (or the repeating units represented by the formula (b1)) is 3 mol% to 80 mol%, preferably 5 mol% to 70 mol%, and more preferably 8 mol% to 65 mol%. The copolymer of the invention may include two or more kinds of repeating units containing a group represented by the formula (b) (or repeating units represented by the formula (b1)).

In the copolymer of the invention, the proportion of the repeating units containing a group represented by the formula (c) (or the repeating units represented by the formula (c1)) may be the balance after deduction of the units having the formulae (a) and (b) from the copolymer, or may be the balance after deduction of the units having the formulae (a) and (b) and a fourth component described later from the copolymer, and is, for example, 1 mol% to 90 mol%, preferably 3 mol% to 88 mol%, and more preferably 5 mol% to 87 mol%. The copolymer of the invention may include two or more kinds of repeating units containing a group represented by the formula (c) (or repeating units represented by the formula (c1)).

The combination of the proportions of the repeating units represented by the formulae (a1), (b1) and (c1) in the copolymer of the invention is preferably:
3 mol% to 80 mol% formula (a1), 3 mol% to 80 mol% formula (b1) and 1 mol% to 90 mol% formula (c1),
   more preferably:
3.5 mol% to 50 mol% formula (a1), 5 mol% to 70 mol% formula (b1) and 3 mol% to 88 mol% formula (c1),
   and still more preferably:
4 mol% to 40 mol% formula (a1), 8 mol% to 65 mol% formula (b1) and 5 mol% to 87 mol% formula (c1).

The copolymer of the invention may further include a desired fourth component. For example, the copolymer may be such that a (meth)acrylate compound having two or more functional groups is copolymerized as a fourth component, and part of the polymer is partially three-dimensionally crosslinked. Examples of such fourth components include bifunctional monomers represented by the following formula (D) or (E):

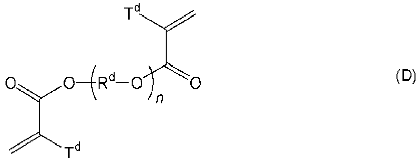

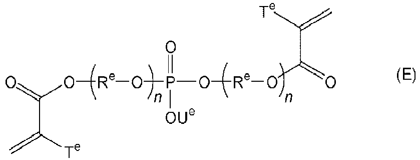

wherein T^{d}, T^{e} and U^{e} are each independently a hydrogen atom or a C₁₋₅ linear or branched alkyl group, R^{d} and R^{e} are each independently an optionally halogenated C₁₋₁₀ linear or branched alkylene group; and n is an integer of 1 to 6. That is, the copolymer according to the present invention preferably includes a crosslinked structure derived from such a bifunctional monomer.

In the formulae (D) and (E), T^{d} and T^{e} are preferably each independently a hydrogen atom, a methyl group or an ethyl group, and more preferably each independently a hydrogen atom or a methyl group.

In the formula (E), U^{e} is preferably a hydrogen atom, a methyl group or an ethyl group, and more preferably a hydrogen atom.

In the formula (D), R^{d} is preferably an optionally halogenated C₁₋₃ linear or branched alkylene group, and is more preferably independently at each occurrence an ethylene or propylene group or an ethylene or propylene group substituted with one chlorine atom, and is particularly preferably an ethylene group or a propylene group. In the formula (D), n is preferably an integer of 1 to 5, and particularly preferably 1.

In the formula (E), R^{e} is preferably an optionally halogenated C₁₋₃ linear or branched alkylene group, and is more preferably independently at each occurrence an ethylene or propylene group or an ethylene or propylene group substituted with one chlorine atom, and is particularly preferably an ethylene group or a propylene group. In the formula (E), n is preferably an integer of 1 to 5, and particularly preferably 1.

Some preferred bifunctional monomers represented by the formula (D) are ethylene glycol di(meth)acrylate, triethylene glycol di(meth)acrylate, propylene glycol di(meth)acrylate, and bifunctional monomers derived from the above formula (A-3).

Some preferred bifunctional monomers represented by the formula (E) are bis(methacryloyloxymethyl) phosphate, bis[(2-methacryloyloxy)ethyl] phosphate, bis[3-(methacryloyloxy)propyl] phosphate, and bifunctional monomers derived from the above formula (A-3).

The optional fourth component may be a trifunctional monomer. Examples of the trifunctional monomers as the fourth components include phosphinylidine tris(oxy-2, 1 -ethanediyl) triacrylate.

Particularly preferred fourth components are ethylene glycol dimethacrylate, dimethacrylates which are bifunctional monomers derived from the formula (A-3) or (A-4) and have ethylene glycol or propylene glycol repeating units, bis[2-(methacryloyloxy)ethyl] phosphate, and dimethacrylates which are bifunctional monomers derived from the formula (A-3) or (A-4) and have ethylene glycol or propylene glycol repeating units via a phosphate group. The structures of these monomers are represented by the following formulae (D-1) to (D-3) and (E-1) to (E-3), respectively.

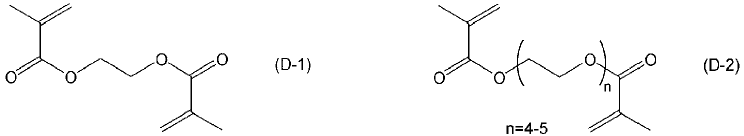

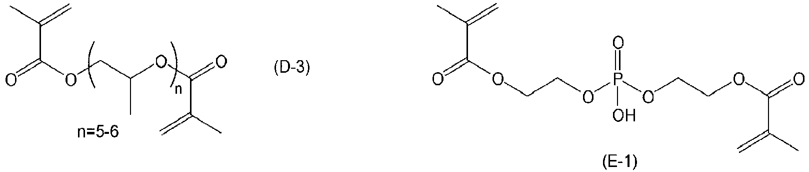

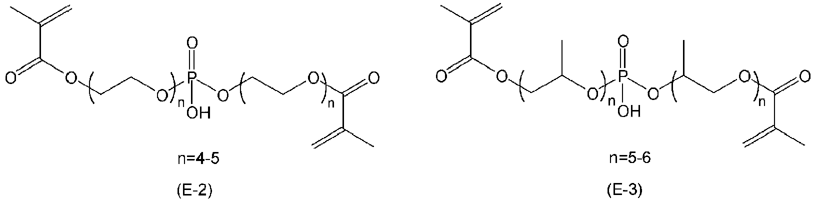

The copolymer may include one, or two or more kinds of fourth components.

In the copolymer, the proportion of the crosslinked structure derived from the fourth component, for example, a bifunctional monomer represented by the formula (D) or (E), is 0 mol% to 50 mol%.

The proportion of the compound represented by the formula (A) relative to all the monomers forming the copolymer is 3 mol% to 80 mol%, preferably 3.5 mol% to 50 mol%, and more preferably 4 mol% to 40 mol%. The copolymer may include two or more kinds of compounds represented by the formula (A).

The proportion of the compound represented by the formula (B) relative to all the monomers forming the copolymer is 3 mol% to 80 mol%, preferably 5 mol% to 70 mol%, and more preferably 8 mol% to 65 mol%. The copolymer may include two or more kinds of compounds represented by the formula (B).

The proportion of the compound represented by the formula (C) relative to all the monomers forming the copolymer may be the balance after deduction of the proportions of the compounds of the formulae (A) and (B), or may be the balance after deduction of the proportions of the compounds of the formulae (A) and (B) and the fourth component, and is, for example, 1 mol% to 90 mol%, preferably 3 mol% to 88 mol%, and more preferably 5 mol% to 87 mol%. The copolymer may include two or more kinds of compounds represented by the formula (C).

The copolymer of the invention may further include, as an optional fifth component, an ethylenically unsaturated monomer, or a polysaccharide or a derivative thereof. The ethylenically unsaturated monomer may be one, or two or more kinds of ethylenically unsaturated monomers selected from the group consisting of (meth)acrylic acid and esters thereof; vinyl acetate; vinylpyrrolidone; ethylene; vinyl alcohols; and hydrophilic functional derivatives thereof. Examples of the polysaccharides and derivatives thereof include cellulose polymers such as hydroxyalkylcelluloses (for example, hydroxyethylcellulose and hydroxypropylcellulose), starch, dextran and curdlan.

The hydrophilic functional derivatives indicate ethylenically unsaturated monomers having a hydrophilic functional group or structure. Examples of the hydrophilic functional groups or structures include betaine structures; amide structures; alkylene glycol residues; amino groups; and sulfinyl groups.

The betaine structures mean monovalent or divalent groups of compounds having an amphoteric center between a quaternary ammonium cation structure and an acid anionic structure, with examples including phosphorylcholine group:

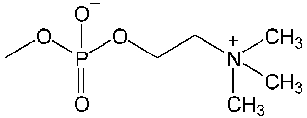

Examples of the ethylenically unsaturated monomers having such a structure include 2-methacryloyloxyethyl phosphorylcholine (MPC).

The amide structures mean groups represented by the following formula: 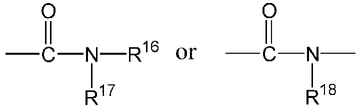 wherein R¹⁶, R¹⁷ and R¹⁸ are each independently a hydrogen atom or an organic group (such as, for example, a methyl group, a hydroxymethyl group or a hydroxyethyl group). Examples of the ethylenically unsaturated monomers having such a structure include (meth)acrylamide and N-(hydroxymethyl) (meth)acrylamide. Further, monomers or polymers having such a structure are disclosed in, for example, Japanese Patent Application Kokai Publication No. 2010-169604.

The alkylene glycol residues mean alkyleneoxy groups (-Alk-O-) which remain after the hydroxyl group(s) at one or both ends of an alkylene glycol (HO-Alk-OH; wherein Alk is a C₁₋₁₀ alkylene group) has condensed with other compound. The residues also include poly(alkyleneoxy) groups having alkyleneoxy repeating units. Examples of the ethylenically unsaturated monomers having such a structure include 2-hydroxyethyl (meth)acrylate and methoxypolyethylene glycol (meth)acrylate. Further, monomers or polymers having such a structure are disclosed in, for example, Japanese Patent Application Kokai Publication No. 2008-533489.

The amino groups mean groups represented by the formula: -NH2, -NHR¹⁹ or -NR²⁰R²¹ wherein R¹⁹, R²⁰ and R²¹ are each independently an organic group (such as, for example, a C₁₋₅ linear or branched alkyl group). The amino groups in the present invention include quaternarized or chlorinated amino groups. Examples of the ethylenically unsaturated monomers having such a structure include dimethylaminoethyl (meth)acrylate, 2-(t-butylamino)ethyl (meth)acrylate and methacryloylcholine chloride.

The sulfinyl groups mean groups represented by the following formula: 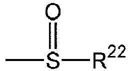 wherein R²² is an organic group (such as, for example, a C₁₋₁₀ organic group, preferably a C₁₋₁₀ alkyl group having one or more hydroxyl groups). Examples of the polymers having such a structure include copolymers disclosed in Japanese Patent Application Kokai Publication No. 2014-48278.

### 〈Copolymer production methods〉

The copolymer of the present invention may be synthesized by a method generally adopted for the synthesis of polymers such as acrylic polymers or methacrylic polymers, with examples including radical polymerization, anionic polymerization and cationic polymerization. The polymerization may be performed in various forms such as solution polymerization, suspension polymerization, emulsion polymerization and bulk polymerization.

The reaction solvent may be water, phosphate buffered solution, alcohol such as ethanol, or a mixture of these solvents, and desirably includes water or ethanol. It is preferable that the solvent include 10 mass% to 100 mass% water or ethanol. It is more preferable that the solvent include 50 mass% to 100 mass% water or ethanol. It is still more preferable that the solvent include 80 mass% to 100 mass% water or ethanol. It is furthermore preferable that the solvent include 90 mass% to 100 mass% water or ethanol. Preferably, the total of water and ethanol is 100 mass% of the solvent.

Regarding the reaction concentration, for example, the concentration of the monomer containing a group represented by the formula (a), the monomer containing a group represented by the formula (b) and the monomer containing a group represented by the formula (c) in the reaction solvent is preferably controlled to 0.01 mass% to 4 mass%. If the concentration is above 4 mass%, for example, the copolymer may be gelled in the reaction solvent due to the strong associative properties of the phosphate groups of the formula (a). If the concentration is below 0.01 mass%, the varnish that is obtained has such a low concentration that it is difficult to prepare a coating film-forming composition which can form a coating film with a sufficient film thickness. The concentration is more preferably 0.01 mass% to 3 mass%, for example, 3 mass% or 2 mass%.

In the synthesis of the copolymer of the invention, a monomer containing a group represented by the formula (a) and a monomer containing a group represented by the formula (b) may be formed into a salt described in, for example, the formula (1) below and the salt may be polymerized together with a monomer containing a group represented by the formula (c) to give a copolymer.

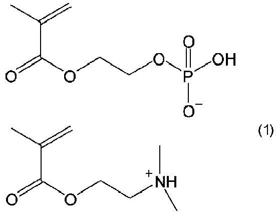

Because the phosphate group-containing monomer is associated easily, it may be added dropwise in small portions to the reaction solvent so that the monomer can be rapidly dispersed after its dropping to the reaction system. The reaction solvent may be heated (for example, 40°C to 100°C) to increase the solubility of the monomers and the polymer.

To allow the polymerization reaction to proceed efficiently, it is preferable to use a polymerization initiator. Examples of the polymerization initiators include 2,2'-azobis(isobutyronitrile), 2,2'-azobis(2-methylbutyronitrile), 2,2'-azobis(2,4-dimethylvaleronitrile) (VA-065 manufactured by Wako Pure Chemical Industries, Ltd., 10 hour half-life temperature: 51°C), 4,4'-azobis(4-cyanovaleric acid), 2,2'-azobis(4-methoxy-2,4-dimethylvaleronitrile), 1,1'-azobis(cyclohexane-1-carbonitrile), 1-[(1-cyano-1-methylethyl)azo]formamide, 2,2'-azobis[2-(2-imidazolin-2-yl)propane], 2,2'-azobis(2-methylpropionamidine) dihydrochloride, 2,2'-azobis[2-methyl-N-(2-hydroxyethyl)propionamide] (VA-086 manufactured by Wako Pure Chemical Industries, Ltd., 10 hour half-life temperature: 86°C), benzoyl peroxide (BPO), 2,2'-azobis[N-(2-carboxyethyl)-2-methylpropionamidine] n-hydrate (VA-057 manufactured by Wako Pure Chemical Industries, Ltd., 10 hour half-life temperature: 57°C), 4,4'-azobis(4-cyanopentanoic acid) (V-501 manufactured by Wako Pure Chemical Industries, Ltd.), 2,2'-azobis[2-(2-imidazolin-2-yl)propane] dihydrochloride (VA-044 manufactured by Wako Pure Chemical Industries, Ltd., 10 hour half-life temperature: 44°C), 2,2'-azobis[2-(2-imidazolin-2-yl)propane] disulfate dihydrate (VA-046B manufactured by Wako Pure Chemical Industries, Ltd., 10 hour half-life temperature: 46°C), 2,2'-azobis[2-(2-imidazolin-2-yl)propane] (VA-061 manufactured by Wako Pure Chemical Industries, Ltd., 10 hour half-life temperature: 61°C), 2,2'-azobis(2-amidinopropane) dihydrochloride (V-50 manufactured by Wako Pure Chemical Industries, Ltd., 10 hour half-life temperature: 56°C), peroxodisulfuric acid and t-butyl hydroperoxide. In light of ion balance and solubility in water, it is preferable to use any of 2,2'-azobis[2-methyl-N-(2-hydroxyethyl)propionamide], 2,2'-azobis(N-(2-carboxyethyl)-2-methylpropionamidine) n-hydrate, 4,4'-azobis(4-cyanopentanoic acid), 2,2'-azobis[2-(2-imidazolin-2-yl)propane] dihydrochloride, 2,2'-azobis[2-(2-imidazolin-2-yl)propane] disulfate dihydrate, 2,2'-azobis[2-(2-imidazolin-2-yl)propane], 2,2'-azobis(2-amidinopropane) dihydrochloride and peroxodisulfuric acid.

The polymerization initiator may be added in an amount of 0.05 mass% to 10 mass% relative to the total weight of the monomers used in the polymerization.

The polymerization reaction may be performed in such a manner that the reaction vessel is heated to 50°C to 200°C in an oil bath or the like, and stirring is performed for 1 hour to 48 hours, or more preferably the reaction vessel is heated to 80°C to 150°C, and stirring is performed for 5 hours to 30 hours. Under such reaction conditions, the copolymer of the invention can be obtained. The reaction atmosphere is preferably a nitrogen atmosphere. Regarding the reaction procedure, all the raw materials may be added to the reaction solvent at room temperature and may be thereafter polymerized while performing heating at the above temperature. Alternatively, a mixture of the raw materials may be added at once or dropwise in small portions to the solvent that has been heated.

When the latter reaction procedure is adopted, for example, a mixture including the compounds of the formulae (A), (B) and (C), a solvent and a polymerization initiator is added dropwise to a solvent kept at a temperature higher than the 10 hour half-life temperature of the polymerization initiator, and the compounds are reacted (polymerized). By adopting such a reaction procedure and temperature conditions, the concentration of the compounds represented by the formula (A), (B) or (C) in the reaction solvent may be controlled to, for example, 0.01 mass% to 10 mass%. In this case, even when the concentration exceeds 4 mass%, the dropped phase and the reaction phase form a transparent uniform solution before the reaction occurs, and consequently the copolymer can be prevented from being gelled in the reaction solvent after the reaction.

The molecular weight of the copolymer of the invention may be about several thousand to several million, preferably 5,000 to 5,000,000, and more preferably 10,000 to 2,000,000. The copolymer may be a random copolymer, a block copolymer or a graft copolymer. The copolymerization reaction for producing the copolymer is not limited, and a known synthesis process performed in a solution such as polymerization utilizing radical polymerization, ion polymerization, photopolymerization or emulsion polymerization may be used. The copolymers according to the present invention may be used singly in accordance with the purpose, or a plurality of copolymers may be mixed in a ratio which may be changed appropriately in accordance with the purpose.

The copolymers produced as described above may be two dimensional polymers or three dimensional polymers, and are in the form of dispersion in an aqueous solution. That is, it is not preferable that the varnish including the polymer be a nonuniform gel or contain whitish precipitates. The varnish is preferably a transparent varnish, a dispersed colloidal varnish or a sol.

The cell culture vessel of the invention is characterized in that at least a portion of the surface of the vessel is coated with the copolymer described hereinabove. It is preferable that the coating be disposed on an inner surface of the vessel which can be brought into contact with cells and culture fluids. It is more preferable that the entire surface of the vessel be coated. The coating of the copolymer may be formed on the cell culture vessel in a known manner, specifically, in the manner described later in 〈〈Methods for producing cell culture vessels〉〉 and Examples. The thickness of the coating on the cell culture vessel of the invention may be selected appropriately in accordance with the shape or purpose of the vessel, but is preferably 10 to 1000 Å, more preferably 10 to 500 Å, and particularly preferably 10 to 300 Å. The cell culture vessel of the present invention has a function of inhibiting adhesion of cells and proteins by virtue of such coating.

### 〈〈Methods for producing cell culture vessels〉〉

The second aspect of the present invention resides in a method for producing a cell culture vessel, including a step of coating at least a portion of the surface of a cell culture vessel with a copolymer that includes a repeating unit containing a group represented by the following formula (a), a repeating unit containing a group represented by the following formula (b), and a repeating unit containing a group represented by the following formula (c):

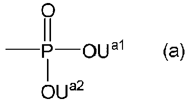

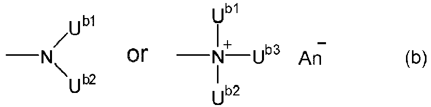

-R^{c} (c)

In the formulae, U^{a1}, U^{a2}, U^{b1}, U^{b2}, U^{b3}, R^{c} and An⁻ are the same as defined hereinabove.

### 〈Coating step〉

In the coating step of the method for producing a cell culture vessel according to the present invention, at least a portion of the surface of a vessel is coated with the copolymer. When, for example, the vessel is a round-bottomed multiwell plate, the coating may be applied only to the holes of the wells or to the entirety of the plate. Here, the vessel and the copolymer are as described in 〈Vessels〉 and 〈Copolymers〉 hereinabove, respectively.

The coating step is not particularly limited and may be performed by a coating technique that is known to the person in the art and can bring the vessel and the copolymer into contact with each other (such as, for example, application or soaking). For example, the coating step may be performed by applying a varnish containing the copolymer to the vessel or by soaking the vessel into a varnish containing the copolymer. Preferably, the coating step is carried out by soaking the vessel into a varnish containing the copolymer.

The varnish containing the copolymer may be prepared by dissolving the copolymer obtained as described in 〈Copolymer production methods〉 hereinabove into an appropriate solvent with a desired concentration. Alternatively, the reaction solution including the copolymer that is obtained by the production method may be used as the varnish as such or after being diluted to a desired solid concentration. Examples of the solvents contained in the varnish include water, phosphate buffered saline (PBS) and alcohols. Examples of the alcohols include C₂₋₆ alcohols such as ethanol, propanol, isopropanol, 1-butanol, 2-butanol, isobutanol, t-butanol, 1-pentanol, 2-pentanol, 3-pentanol, 1-heptanol, 2-heptanol, 2,2-dimethyl-1-propanol (= neopentyl alcohol), 2-methyl-1-propanol, 2-methyl-1-butanol, 2-methyl-2-butanol (= t-amyl alcohol), 3-methyl-1-butanol, 3-methyl-3-pentanol, cyclopentanol, 1-hexanol, 2-hexanol, 3-hexanol, 2,3-dimethyl-2-butanol, 3,3-dimethyl-1-butanol, 3,3-dimethyl-2-butanol, 2-ethyl-1-butanol, 2-methyl-1-pentanol, 2-methyl-2-pentanol, 2-methyl-3-pentanol, 3-methyl-1-pentanol, 3-methyl-2-pentanol, 3-methyl-3-pentanol, 4-methyl-1-pentanol, 4-methyl-2-pentanol, 4-methyl-3-pentanol and cyclohexanol. The solvents may be used singly, or may be combined into a mixed solvent. It is preferable that the solvent be selected from water, PBS and ethanol. The solvent necessarily includes water to dissolve the copolymer.

The concentration of the copolymer in the varnish is 0.01 to 4 mass%, more desirably 0.01 to 3 mass%, still more desirably 0.01 to 2 mass%, and further desirably 0.01 to 1 mass%. If the concentration of the copolymer is below 0.01 mass%, the varnish fails to form a coating film with a sufficient film thickness. If the concentration is above 4 mass%, the storage stability of the varnish is deteriorated and there is a risk that the solutes may be precipitated or the varnish may be gelled.

In addition to the copolymer and the solvent, the varnish may include other materials as required without impairing the performance of the coating films that are obtained. Examples of such additional materials include preservatives, surfactants, primers for enhancing the adhesion to the base (the vessel), antifungal agents and sugars.

To control the ion balance of the copolymer in the varnish, the pH of the varnish containing the copolymer may be adjusted beforehand. The pH may be adjusted by, for example, adding a pH adjuster to the varnish containing the copolymer to control the pH of the varnish to 3.5 to 8.5, or preferably 4.0 to 8.0. The type and amount of the pH adjuster may be selected appropriately in accordance with factors such as the concentration of the copolymer in the varnish and the ratio of anions and cations in the copolymer. Examples of the pH adjusters include organic amines such as ammonia, diethanolamine, pyridine, N-methyl-D-glucamine and tris(hydroxymethyl)aminomethane; alkali metal hydroxides such as potassium hydroxide and sodium hydroxide; alkali metal halides such as potassium chloride and sodium chloride; inorganic acids such as sulfuric acid, phosphoric acid, hydrochloric acid and carbonic acid, and alkali metal salts thereof; quaternary ammonium cations such as choline; and mixtures thereof (for example, buffers such as phosphate buffered physiological saline). Of these, ammonia, diethanolamine, N-methyl-D-glucamine, tris(hydroxymethyl)aminomethane, sodium hydroxide and choline are preferable, and ammonia, diethanolamine, sodium hydroxide and choline are particularly preferable.

The varnish containing the copolymer that is described above is brought into contact with the vessel so as to form a coating on at least a portion of the surface of the vessel. It is preferable that the coating be formed over the entire surface of the vessel.

Before the coating step, the surface of the vessel may be cleaned by known UV/ozone treatment. Such cleaning step may be performed using, for example, a commercial UV/ozone cleaner.

### 〈Drying, washing and sterilizing steps〉

After the coating step, the coated vessel is preferably dried at a temperature in the range of -200°C to 200°C. The drying removes the solvent in the coating film-forming composition, and causes the copolymer of the invention to form ion bonds between the formula (a) and the formula (b), resulting in complete adhesion to the base. The film thickness of the coating on the cell culture vessel of the invention is preferably 10 to 1000 Å, more preferably 10 to 500 Å, and particularly preferably 10 to 300 Å. The present inventors have found that the method for producing cell culture vessels of the present invention can form a coating with the desired properties on the surface of the vessel without the need of a high-temperature treatment, i.e., by the treatment at a low temperature, and the coating has outstanding durability in spite of the thickness being as small as about several tens to several hundreds of Å and also has a function of inhibiting adhesion of cells and proteins.

The drying may be performed at, for example, room temperature (10°C to 35°C, for example, 25°C). To promote the formation of the coating film, the drying may be performed at, for example, 40°C to 50°C. The drying may be performed by a freeze drying method at a very low or low temperature (about -200°C to -30°C).
Freeze drying is also called vacuum freeze drying, and is usually performed by freezing a wet product with a refrigerant and removing the solvent by sublimation under vacuum. Some example refrigerants generally used in the freeze drying are a mixed medium of dry ice and methanol (-78°C), and liquid nitrogen (-196°C). The drying temperature is more preferably 10°C to 180°C, and still more preferably 25°C to 150°C.

Before and/or after the drying step, the surface of the coated vessel may be washed with a C₁₋₅ alcohol such as ethanol and/or water. The washing step may be performed at a temperature of 0°C to 60°C, preferably 25°C (room temperature) to 40°C, for 30 minutes to 48 hours, preferably 1 to 24 hours.

To remove undesired components such as impurities and unreacted monomers remaining in the coating film, and to adjust the ion balance of the copolymer in the film, the coating film after the drying step may be washed with at least one solvent selected from the group consisting of water and aqueous electrolyte solutions by a method such as running or ultrasonication of the solvent. The water or the aqueous electrolyte solution may be heated beforehand in the range of, for example, 40°C to 95°C. Some preferred aqueous electrolyte solutions are PBS, physiological saline (containing only sodium chloride), Dulbecco's phosphate buffered physiological saline, Tris buffered physiological saline, HEPES buffered physiological saline and Veronal buffered physiological saline, with PBS being particularly preferable.

The coating formed on the surface of the vessel remains firmly fixing to the base (the vessel) without eluting even when washed with alcohol, water, PBS or the like. The coating has a function of inhibiting adhesion of various biomaterials including cells and proteins. Thus, the cell culture vessel of the present invention has a function of inhibiting adhesion of cells and proteins and is highly durable in contact with solvents.

Where necessary, the coated vessel may be sterilized by a known sterilization treatment using, for example, radiation, electron beam, ethylene oxide or an autoclave.

### 〈〈Methods for producing cell aggregates〉〉

The third aspect of the present invention resides in a method for producing a cell aggregate, characterized by using the cell culture vessel of the present invention, or a cell culture vessel obtained by the production method of the present invention (hereinbelow, both cell culture vessels will be collectively written as the "cell culture vessel of the invention"). Because the cell culture vessel of the invention can inhibit the adhesion of cell aggregates to the surface of the vessel, and also because the coating is hardly eluted into the culture fluid, the use of the cell culture vessel allows cell aggregates to be cultured in the absence of stimuli from the vessel. The cell aggregates are preferably cultured using a medium characterized by containing a polysaccharide (in particular, deacylated gellan gum) which can effectively keep cells or tissues suspended in the cell culture vessel of the invention. The specific compositions of such media and the specific culture methods are disclosed in, for example, WO 2014/017513.

### EXAMPLES

Hereinbelow, the culture vessels of the present invention and the methods for the production thereof will be described based on Synthetic Examples, Examples and Test Examples related thereto. The following discussion is only illustrative of the further details of the present invention and does not intend to limit the scope of the invention thereto.

### 〈Method for measuring proportions of raw materials〉

The respective concentrations (mass%) of phosphorus-containing compounds in raw materials were determined by ³¹P-NMR. The absolute concentration (absolute mass%) of each phosphorus-containing compound in raw materials was calculated using the standard substance described below.

### (Measurement conditions)

- Mode: inverse gated decoupling mode (quantitative mode)
- Device: Varian 400 MHz
- Solvent: CD₃OD (deuterated methanol) (30 wt%)
- Rotational frequency: 0 Hz
- Data points: 64000
- Flip angle: 90°
- Waiting time: 70 s
- Scans: 16, n = 4
- Standard substance: trimethyl phosphate + D₂O (prepared as 75% TMP solution)

### 〈Synthetic Example 1〉

16.05 g of pure water was added to 7.00 g of acid phosphoxyethyl methacrylate (product name: Phosmer M manufactured by Uni-Chemical Co., nonvolatile content measured by drying process at 100°C for 1 hour: 91.8%, mixture of acid phosphoxyethyl methacrylate (44.2 mass%), bis[2-(methacryloyloxy)ethyl] phosphate (28.6 mass%) and others (27.2 mass%)), and the compound was sufficiently dissolved. Next, while keeping the temperature at not more than 20°C, 48.16 g of ethanol, 9.41 g of 2-(diethylamino)ethyl methacrylate (manufactured by Tokyo Chemical Industry Co., Ltd.), 1.34 g of butyl methacrylate (manufactured by Tokyo Chemical Industry Co., Ltd.) and 0.09 g of 2,2'-azobis(N-(2-carboxyethyl)-2-methylpropionamidine) n-hydrate (VA-057 manufactured by Wako Pure Chemical Industries, Ltd.) were added sequentially to the aqueous solution of Phosmer M. The resultant mixture was sufficiently stirred to uniformity. The mixture liquid thus obtained which contained the above materials was introduced into a dropping funnel. Separately, 96.31 g of pure water was added to a three-necked flask having a cooling tube. While flowing nitrogen into the flask and while performing stirring, the temperature was increased to reflux temperature. While maintaining this state, the dropping funnel containing the mixture liquid was fitted to the three-necked flask, and the mixture liquid was dropped to the boiling mixture of pure water and ethanol over a period of 2 hours. After the completion of the dropwise addition, the system was heated and stirred for 24 hours while maintaining the above environment. Consequently, 178.35 g of a copolymer-containing varnish having a solid content of about 10 mass% was obtained.

### 〈Comparative Synthetic Example 1〉

While keeping the temperature at 60°C, 28.00 g of acid phosphoxyethyl methacrylate (product name: Phosmer M manufactured by Uni-Chemical Co., nonvolatile content measured by drying process at 100°C for 1 hour: 91.8%, mixture of acid phosphoxyethyl methacrylate (44.2 mass%), bis[2-(methacryloyloxy)ethyl] phosphate (28.6 mass%) and others (27.2 mass%)) was stirred, and 21.37 g of 2-(dimethylamino)ethyl methacrylate was added thereto dropwise. While keeping the temperature at not more than 20°C, 133.96 g of pure water, then 44.65 g of ethanol (manufactured by Tokyo Chemical Industry Co., Ltd.) and 0.25 g of 2,2'-azobis(N-(2-carboxyethyl)-2-methylpropionamidine) n-hydrate (VA-057 manufactured by Wako Pure Chemical Industries, Ltd.) were added to the mixture sequentially. The resultant mixture was sufficiently stirred to uniformity. The mixture liquid thus obtained which contained the above materials was introduced into a dropping funnel. Separately, 267.93 g of pure water was added to a three-necked flask having a cooling tube. While flowing nitrogen into the flask and while performing stirring, the temperature was increased to reflux temperature. While maintaining this state, the dropping funnel containing the mixture liquid was fitted to the three-necked flask, and the mixture liquid was dropped to the boiling mixture of pure water and ethanol over a period of 2 hours. After the completion of the dropwise addition, the system was heated and stirred for 24 hours while maintaining the above environment. Consequently, 496.16 g of a copolymer-containing varnish having a solid content of about 9.70 mass% was obtained. GFC analysis of the transparent liquid obtained showed that the weight average molecular weight was about 280,000.

### 〈Preparation Example 1〉

### (Provision of silicon wafers)

Commercial silicon wafers for semiconductor evaluation were used directly.

### (Preparation of QCM sensor (PS))

An Au-deposited quartz crystal oscillator (Q-Sense, QSX304) was cleaned for 10 minutes using a UV/ozone cleaning device (UV253E, manufactured by Filgen, Inc.). Immediately thereafter, it was immersed into a solution of 0.0772 g of 2-aminoethanethiol (manufactured by Tokyo Chemical Industry Co., Ltd.) in 1000 ml of ethanol for 24 hours. The surface of the sensor was washed with ethanol and was allowed to dry naturally. The film sensor side was spin coated with a varnish obtained by dissolving 1.00 g of polystyrene (PS) (manufactured by Aldrich Corporation) in 99.00 g of toluene by use of a spin coater at 3500 rpm for 30 seconds, and the coating was dried at 120°C for 1 minute. A QCM sensor (PS) was thus obtained.

### (Preparation of coating)

31.5 g of pure water, 1.35 g of ethanol, and 0.24 g of a 1 mol/L aqueous sodium hydroxide solution (1 N) (manufactured by KANTO CHEMICAL CO., INC.) were added to 5.00 g of the copolymer-containing varnish obtained in Synthetic Example 1. The mixture was sufficiently stirred to give a coating film-forming composition. The pH was 7.3. The silicon wafer was dipped into the coating film-forming composition and was dried in an oven at 45°C for 24 hours. Thereafter, the uncured film-forming composition residing on the coating film was sufficiently removed by washing with PBS and pure water. Thus, a coating film was formed on the silicon wafer. The silicon wafer was analyzed with an optical interferometric thickness gauge, and the film thickness of the coating film was measured to be 90 Å.

Further, the coating film-forming composition was spin coated onto the QCM sensor (PS) at 3500 rpm for 30 seconds and was subjected to a drying step in which the film was baked in an oven at 45°C for 24 hours. Thereafter, a washing step was performed in which PBS and ultrapure water were applied each two times to remove the uncured excess portion of the coating film-forming composition. A surface-treated QCM sensor (PS) was thus obtained.

### 〈Preparation Example 2〉

A QCM sensor (PS) similar to that described above was used as such.

### 〈Preparation Example 3〉

265.92 g of pure water, 125.58 g of ethanol, and 8.18 g of a 1 mol/L aqueous sodium hydroxide solution (1 N) (manufactured by KANTO CHEMICAL CO., INC.) were added to 45.00 g of the copolymer-containing varnish obtained in Comparative Synthetic Example 1. The mixture was sufficiently stirred to give a coating film-forming composition. A silicon wafer having a coating film, and a surface-treated QCM sensor (PS) were obtained in the same manner as in Example. The silicon wafer was analyzed with an optical interferometric thickness gauge, and the film thickness of the coating film was measured to be 44 Å.

### (Test Example 1: a function of inhibiting adhesion of proteins)

The surface-treated QCM sensors (PS) obtained in Preparation Examples 1 to 3 were each fitted to quartz crystal microbalance with dissipation monitoring QCM-D (E4, Q-Sense), and PBS was passed until a stable baseline was achieved in which the change in frequency in one hour was 1 Hz or less. Next, the frequency of the stable baseline was taken as 0 Hz and PBS was passed for about 10 minutes. Subsequently, a solution in which 15 wt% fetal bovine serum (FBS), L-Glutamine, and penicillin and streptomycin as antibiotics had been added to 41010 - Basal Medium Eagle (BME), no Glutamine (available from Thermo Fisher Scientific Inc.), was passed for about 30 minutes. Thereafter, PBS was passed again for about 20 minutes, and the adsorption induced shift in frequency (Δf) of the eleventh overtone was read. Using Q-Tools (Q-Sense), the adsorption induced frequency shift (Δf) was analyzed based on the Sauerbrey equation which explains the conversion of the adsorption induced frequency shift (Δf) to the mass change per unit area (ng/cm²), the results being shown in Table 1 below as the amounts of biomaterial attachment. Preparation Example 1 which involved a coating of the copolymer of Synthetic Example 1 in accordance with the present invention attained a reduction by one order of magnitude in the amount of protein adsorption compared to Preparation Example 2 in which no coating was formed and Preparation Example 3 which involved a coating of the copolymer of Comparative Synthetic Example 1.

**[Table 1]**

| | Coating | Amount of protein adhesion (ng/cm²) |
|---|---|---|
| Preparation Example 1 | Copolymer of Synthetic Example 1 | 16 |
| Preparation Example 2 | No coating (PS surface) | 566 |
| Preparation Example 3 | Copolymer of Comparative Synthetic Example 1 | 297 |

### 〈Example 1〉

The following treatment steps were performed sequentially to prepare a cell culture vessel of the present invention.

Treatment 1: The copolymer-containing varnish prepared in Synthetic Example 1 was filtered through a filter having a mesh size of 0.22 µm, and was then added to wells of a 96-well cell culture plate (#351172 manufactured by BD Biosciences) so that each well contained 200 µL varnish (1 mass% solid content). The plate was allowed to stand at room temperature for 1 hour and was cleaned of excess varnish.

Treatment 2: The varnish was dried in an oven (dryer FC-612 manufactured by Advantec Toyo Kaisha, Ltd.) at 50°C overnight. Thereafter, 200 µL of sterilized water was added per well and was removed, thereby washing the wells. This washing was performed another two times. A coated cell culture vessel was thus obtained.

### 〈Comparative Example 1〉

A coated cell culture vessel was obtained in the same manner as in Example 1, except that the copolymer-containing varnish from Synthetic Example 1 that was used in Example 1 was replaced by the copolymer-containing varnish from Comparative Synthetic Example 1.

### 〈Test Example 2: a function of inhibiting adhesion of cells〉

### (Preparation of coated plates)

The coated cell culture vessels obtained in Example 1 and Comparative Example 1 were used. As a positive control sample, a commercial low cell adhesion plate (#3474 manufactured by Corning Inc.) was used. As a negative control, an uncoated 96-well cell culture plate (#351172 manufactured by BD Biosciences) was used.

### (Preparation of cells)

Mouse embryonic fibroblasts C3H10T1/2 (available from DS Pharma Biomedical Co., Ltd.) were used. The medium used for the culturing of the cells was BME medium (available from Thermo Fisher Scientific Inc.) containing 10% FBS (available from HyClone Laboratories, Inc.) and L-glutamine-penicillin-streptomycin stabilized solution (available from SIGMA-ALDRICH Co. LLC.). The cells were statically cultured for at least 2 days in a 10 cm-diameter petri dish (10 mL culture medium) in a CO₂ incubator at 37°C while keeping 5% carbon dioxide concentration. Subsequently, the cells were washed with 5 mL of PBS, and thereafter 1 mL of trypsin-EDTA solution (available from Invitrogen Co.) was added thereto to separate the cells, which were then each suspended in 10 mL of the above-mentioned medium. The suspensions were centrifuged (model No. LC-200 manufactured by TOMY SEIKO CO., LTD., 1000 rpm/3 min, room temperature), the supernatant was removed, and the above-mentioned medium was added. Cell suspensions were thus prepared.

### (Cell adhesion experiment)

To the plates, the cell suspensions were added in a volume of 100 µL so that each well would contain 2 x 10³ cells/well. Thereafter, the plates were allowed to stand in a CO₂ incubator at 37°C for 4 days while keeping 5% carbon dioxide concentration.

### (Observation for cell adhesion)

After 4 days of culturing, the cell adhesion was compared among the plate of Example 1, the plate of Comparative Example 1, the positive control plate and the negative control plate based on observation (magnification: x4) with an inverted microscope (CKX31 manufactured by Olympus Corporation). Substantially no cell adhesion was seen in the plate of Example 1, the plate of Comparative Example 1 and the positive control plate. The results (after 4 days of culturing) of the plates are shown in Fig. 1. Further, Cell Counting Kit-8 solution (available from DOJINDO LABORATORIES) was added in 10 µL per well, and the plates were allowed to stand in a CO₂ incubator at 37°C for 2 hours. Thereafter, the absorbance at 450 nm was measured with an absorptiometer (SpectraMax manufactured by Molecular Devices, LLC.). The absorbance measured with respect to a well containing only the medium was subtracted from each of the values that were measured. The results are described in Table 2.

**[Table 2]**

| Absorbance measurement | |
|---|---|
| | Absorbance |
| Example 1 | 0.061 |
| Comparative Example 1 | 0.053 |
| Positive control | 0.034 |
| Negative control | 0.511 |

As shown above, all the plates except the negative control were free from cell adhesion. In these plates, the cells had formed cell aggregates (spheroids).

### Industrial Applicability

The cell culture vessels of the present invention are coated, on at least a portion of the surface thereof, with a copolymer containing specific groups. Such a coating inhibits the adhesion of cells and proteins to the surface of the vessel, and is firmly fixed onto the surface of the vessel and negligibly eluted into a culture fluid. Thus, the cell culture vessels of the invention are advantageous in that cell aggregates can be cultured therein without stimuli from the vessel.

## Claims

1. A cell culture vessel having a surface coated with a copolymer comprising a repeating unit containing a group represented by the following formula (a), a repeating unit containing a group represented by the following formula (b), and a repeating unit containing a group represented by the following formula (c): 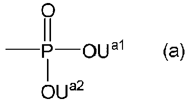 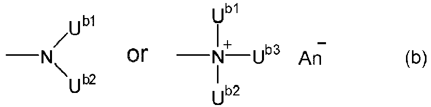
-R^{c} (c)
wherein
U^{a1}, U^{a2}, U^{b1}, U^{b2} and U^{b3} are each independently a hydrogen atom or a C₁₋₅ linear or branched alkyl group;
R^{c} is a C₁₋₁₈ linear or branched alkyl group, a C₃₋₁₀ alicyclic hydrocarbon group, a C₆₋₁₀ aryl group, a C₇₋₁₅ aralkyl group or a C₇₋₁₅ aryloxyalkyl group, wherein the aryl moiety may be substituted with an optionally halogenated C₁₋₅ linear or branched alkyl group; and
An⁻ is an anion selected from the group consisting of halide ion, inorganic acid ion, hydroxide ion and isothiocyanate ion.

2. The cell culture vessel according to Claim 1, wherein the repeating units containing groups represented by the formulae (a), (b) and (c) are repeating units derived from monomers represented by the following formulae (A), (B) and (C), respectively: 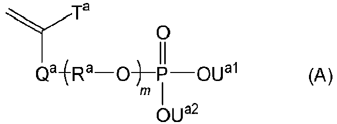 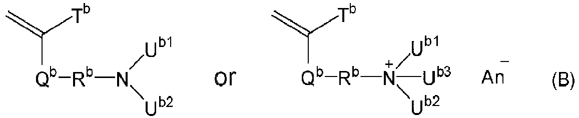 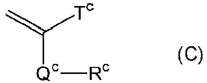 wherein
T^{a}, T^{b}, T^{c}, U^{a1}, U^{a2}, U^{b1}, U^{b2} and U^{b3} are each independently a hydrogen atom or a C₁₋₅ linear or branched alkyl group;
Q^{a} and Q^{b} are each independently a single bond, an ester bond or an amide bond, Q^{c} is a single bond, an ether bond or an ester bond;
R^{a} and R^{b} are each independently an optionally halogenated C₁₋₁₀ linear or branched alkylene group, R^{c} is a C₁₋₁₈ linear or branched alkyl group, a C₃₋₁₀ alicyclic hydrocarbon group, a C₆₋₁₀ aryl group, a C₇₋₁₅ aralkyl group or a C₇₋₁₅ aryloxyalkyl group, wherein the aryl moiety may be substituted with an optionally halogenated C₁₋₅ linear or branched alkyl group; An⁻ is an anion selected from the group consisting of halide ion, inorganic acid ion, hydroxide ion and isothiocyanate ion; and
m is an integer of 0 to 6.

3. The cell culture vessel according to Claim 2, wherein m is 1, and R^{a} and R^{b} are each independently an ethylene group or a propylene group.

4. The cell culture vessel according to any one of Claims 1 to 3, wherein the copolymer further comprises a crosslinked structure derived from a monomer represented by the following formula (D) or (E): 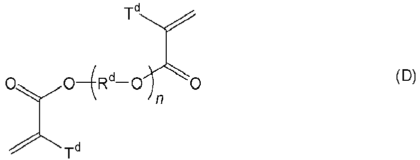 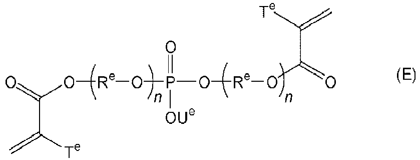 wherein
T^{d}, T^{e} and U^{e} are each independently a hydrogen atom or a C₁₋₅ linear or branched alkyl group;
R^{d} and R^{e} are each independently an optionally halogenated C₁₋₁₀ linear or branched alkylene group; and
n is an integer of 1 to 6.

5. The cell culture vessel according to Claim 4, wherein T^{d} and T^{e} are each independently a hydrogen atom or a methyl group, U^{e} is a hydrogen atom, and R^{d} and R^{e} are each independently an ethylene group or a propylene group.

6. The cell culture vessel according to any one of Claims 1 to 5, which has a function of inhibiting adhesion of proteins.

7. A method for producing a cell culture vessel, comprising a step of coating at least a portion of the surface of a cell culture vessel with a copolymer comprising a repeating unit containing a group represented by the following formula (a), a repeating unit containing a group represented by the following formula (b), and a repeating unit containing a group represented by the following formula (c): 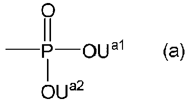 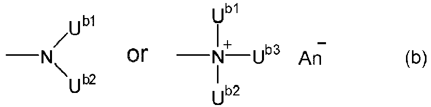
-R^{c} (c)
wherein
U^{a1}, U^{a2}, U^{b1}, U^{b2} and U^{b3} are each independently a hydrogen atom or a C₁₋₅ linear or branched alkyl group;
R^{c} is a C₁₋₁₈ linear or branched alkyl group, a C₃₋₁₀ alicyclic hydrocarbon group, a C₆₋₁₀ aryl group, a C₇₋₁₅ aralkyl group or a C₇₋₁₅ aryloxyalkyl group, wherein the aryl moiety may be substituted with an optionally halogenated C₁₋₅ linear or branched alkyl group; and
An⁻ is an anion selected from the group consisting of halide ion, inorganic acid ion, hydroxide ion and isothiocyanate ion.

8. The production method according to Claim 7, wherein the repeating units containing groups represented by the formulae (a), (b) and (c) are repeating units derived from monomers represented by the following formulae (A), (B) and (C), respectively: 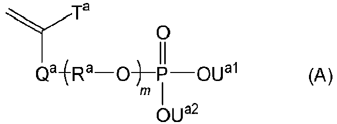 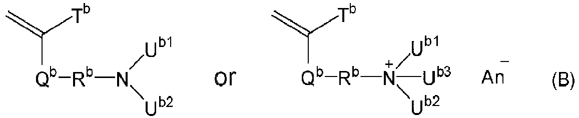 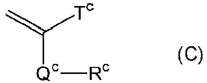 wherein
T^{a}, T^{b}, T^{c}, U^{a1}, U^{a2}, U^{b1}, U^{b2} and U^{b3} are each independently a hydrogen atom or a C₁₋₅ linear or branched alkyl group;
Q^{a} and Q^{b} are each independently a single bond, an ester bond or an amide bond, Q^{c} is a single bond, an ether bond or an ester bond;
R^{a} and R^{b} are each independently an optionally halogenated C₁₋₁₀ linear or branched alkylene group, R^{c} is a C₁₋₁₈ linear or branched alkyl group, a C₃₋₁₀ alicyclic hydrocarbon group, a C₆₋₁₀ aryl group, a C₇₋₁₅ aralkyl group or a C₇₋₁₅ aryloxyalkyl group, wherein the aryl moiety may be substituted with an optionally halogenated C₁₋₅ linear or branched alkyl group; An⁻ is an anion selected from the group consisting of halide ion, inorganic acid ion, hydroxide ion and isothiocyanate ion; and
m is an integer of 0 to 6.

9. The production method according to Claim 7 or 8, wherein the coating step is performed using a varnish including the copolymer.

10. The production method according to Claim 9, wherein the pH of the varnish including the copolymer is adjusted beforehand.

11. The production method according to any one of Claims 7 to 10, further comprising a step of washing the cell culture vessel coated, before and/or after a drying step.

12. The production method according to Claim 11, wherein the washing after the drying step is performed using at least one solvent selected from the group consisting of water and aqueous electrolyte solutions.

13. A method for producing a cell aggregate, **characterized by** using the cell culture vessel described in any one of Claims 1 to 6, or a cell culture vessel produced by the production method described in any one of Claims 7 to 12.
